(19) Europäisches Patentamt
European Patent Office
Office européen des brevets

(11) Publication number: 0 567 643 A1

(12) # EUROPEAN PATENT APPLICATION published in accordance with Art. 158(3) EPC

(21) Application number: 91902750.8

(22) Date of filing: 16.01.91

(86) International application number: PCT/JP91/00037

(87) International publication number: WO 92/12976 (06.08.92 92/21)

(51) Int. Cl.5: C07D 401/12, C07D 401/14, C07D 405/14, C07D 409/14, C07D 413/14, C07D 417/14, A61K 31/44, A61K 31/535, A61K 31/54

(43) Date of publication of application: 03.11.93 Bulletin 93/44

(84) Designated Contracting States: AT BE CH DE DK ES FR GB GR IT LI LU NL SE

(71) Applicant: **YOSHITOMI PHARMACEUTICAL INDUSTRIES, LTD.**
**6-9, Hiranomachi 2-chome**
**Chuo-ku**
**Osaka-shi Osaka 541(JP)**

(72) Inventor: **KAWAKITA, Takeshi 7-8, Chuomachi 1-chome**
**Nakatsu-shi**
**Oita 871(JP)**
Inventor: **SANO, Mitsuharu 276-2, Yatsunami**
**Shinyoshitomimura**
**Chikuyo-gun Fukuoka 871-09(JP)**
Inventor: **IKEDA, Yoshifumi 914-2,**
**Oaza-Nagasoe**
**Nakatsu-shi**
**Oita 871(JP)**
Inventor: **IWAO, Eiji 12-6, Toyodacho**
**Nakatsu-shi**
**Oita 871(JP)**
Inventor: **HAGA, Keiichiro 10-76, Chuomachi 1-chome**
**Nakatsu-shi**
**Oita 871(JP)**

(74) Representative: **von Kreisler, Alek, Dipl.-Chem. et al**
**Patentanwälte,**
**Von Kreisler-Selting-Werner,**
**Postfach 10 22 41,**
**Bahnhofsvorplatz 1**
**D-50462 Köln (DE)**

(54) **USE OF PYRIDINE COMPOUND AS SELECTIVE DRUG AND NOVEL PYRIDINE COMPOUND.**

(57) A compound of general formula (I) and its salt, wherein each symbol is as defined in the specification, and a new compound of general formula (II) and its salt which are included in the above compound and its salt. These compounds have an excellent selective sterilization effect on the bacteria belonging to the genus Helicobacter and are useful for preventing the recrudescence and recidivation of ulcers, suppressing vomit, preventing and treating non-ulcer dyspepsia, preventing and treating tumors, and also as an antiulcer drug.

EP 0 567 643 A1

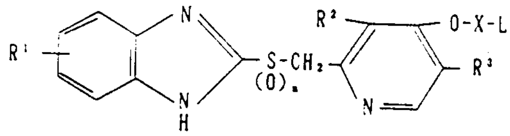

(I)

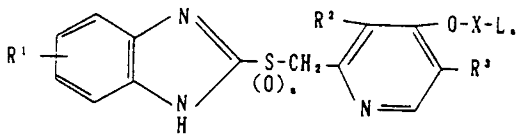

(II)

## TECHNICAL FIELD

The present invention relates to a use of a novel or known pyridine compound as a selective drug, and to a novel pyridine compound useful for the prevention and treatment of disorders in the digestive tract.

## BACKGROUND ART

The bacterium belonging to the genus *Campylobacter* which is a Gram-negative microaerophile was found as a bacterium causing diarrhea and miscarriage in domestic animals. With regard to humans, there have been known *Campylobacter jejuni* and *Campylobacter coli* as bacteria causing bacterial enteritis. In 1983, Warren et al. reported involvement of the infection of *Campylobacter pylori* (current name: *Helicobacter pylori*, hereinafter the current name is used) in gastritis, and there have been done a multitude of studies and presented many reports ever since. Actually, *Helicobacter pylori* has been found, at high frequencies, in antral gastritis tissues to be combined with chronic gastritis, or gastric ulcer and duodenal ulcer.

In recent years, there are increasing numbers of reports teaching that duodenal ulcer, chronic gastritis, and non-ulcer dyspepsia can be healed by the antibacterial action on *Helicobacter pylori*, and that recurrence and relapse of the diseases are few. For example, of the 73 patients out of the 75 patients with relapsed or resistant duodenal ulcer, who were initially treated with ranitidine and then with triple administration of colloidal bismuth subcitrate, tetracycline, and metronidazole for 4 weeks, 71 patients did not suffer from infection of *Helicobacter pylori* or relapse of the ulcer at 1 year later. In the second year, all 57 patients who underwent endoscopy did not carry the bacterium nor did they suffer from relapse of the ulcer. At three years from the treatment, 34 patients underwent an examination, and the bacterium was not found in 33 of them, with no evidence of relapse of duodenal ulcer in all of the 34 patients. In contrast, the percent relapse in case of the sole treatment with ranitidine was 70-90% (SCRIP, No. 1549, p. 24, Sep. 14, 1990). The Lancet, vol. 335, p. 1599 (1990) reports that of the 48 child patients treated with antibiotics to exert antibacterial action on *Helicobacter pylori*, only one child suffered from relapsed duodenal ulcer after the average two-year follow up examination, and the patients who did not suffer from relapse carried no *Helicobacter pylori*. The Lancet, vol. 336, p. 755 (1990) reports the results of a 4-week treatment of 61 *Helicobacter pylori*-positive, duodenal ulcer patients, who were divided into two groups (a group administered with ranitidine and a group combinedly administered with ranitidine and oxacillin), in terms of percent relapse after 12 months, wherein the group administered with ranitidine showed 86% of relapse (*Helicobacter pylori* tested positive in all patients) and the group administered combinedly showed 37% of relapse (*Helicobacter pylori* tested positive in 16 patients out of 32). Further, The Lancet, No. 8626/8627, p. 1502 (1988) reports that the administration of antacid, metoclopramide, domperidone, etc. was ineffective against vomiting observed in the patients with *Helicobacter pylori*-related gastritis, whereas antibacterial treatment on *Helicobacter pylori* could suppress the vomiting. Also, *Helicobacter pylori* is said to induce tumor.

It should be noted that the bismuth compounds have low potency in the antibacterial action on *Helicobacter pylori*, and accompany vomiting and central side effects. In addition, antibiotics exert influence on other bacteria as well, and the administration on a long term basis is not desirable.

As the situation stands, development of a useful pharmaceutical usable for the prophylaxis and treatment of various diseases caused by *Helicobacter pylori* has been demanded. For inhibiting recurrence and relapse of ulcer, as well as suppression of vomiting, a novel pharmaceutical having antiulcer activity, gastric acid secretion-suppressing activity, gastrointestinal cell-protecting activity, and antidiarrhea activity besides the antibacterial activity on the bacteria belonging to the genus *Helicobacter*, has a great demand for development.

## DISCLOSURE OF THE INVENTION

The present invention is as follows.

(1) An agent for the prevention and treatment of various diseases caused by the bacteria belonging to the genus *Helicobacter*, comprising a pyridine compound of the formula

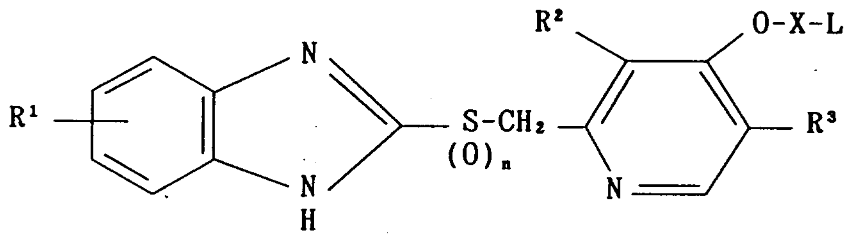

(I)

wherein $R^1$ is hydrogen, halogen, alkyl, alkoxy, alkoxycarbonyl, or haloalkyl, $R^2$ and $R^3$ are the same or different and each is hydrogen, halogen or alkyl, n is 0, 1 or 2, X is single bond or alkylene, and L is alkoxy, a group of the formula

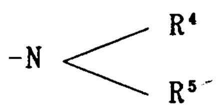

wherein $R^4$ and $R^5$ are the same or different, and each is hydrogen, alkyl, acyl, phenyl, substituted phenyl, phenylalkyl, substituted phenylalkyl, or furylalkyl, thienylalkyl or pyridylalkyl which may be substituted, or $R^4$ and $R^5$ together with the adjacent nitrogen atom form a heterocyclic ring which may be condensed, or a group of the formula

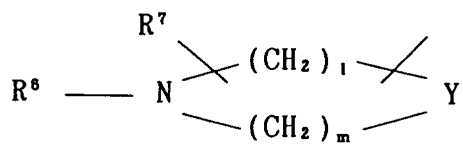

wherein $R^6$ is hydrogen, alkyl, acyl, phenylalkyl, or substituted phenylalkyl, $R^7$ is hydrogen, halogen, alkyl, alkoxy, haloalkyl, phenyl, substituted phenyl, phenylalkyl, substituted phenylalkyl, heteroarylalkyl, or substituted heteroarylalkyl, Y is methylene, oxygen or sulfur, and l and m are the same or different and each is 0, 1, 2 or 3, or a pharmaceutically acceptable salt thereof.

(2) An agent for inhibiting recurrence and relapse of ulcer, a suppressant of vomiting, or an agent for the prevention and treatment of non-ulcer dyspepsia, comprising the compound of the formula (I) or a pharmaceutically acceptable salt thereof.

(3) A pyridine compound of the formula

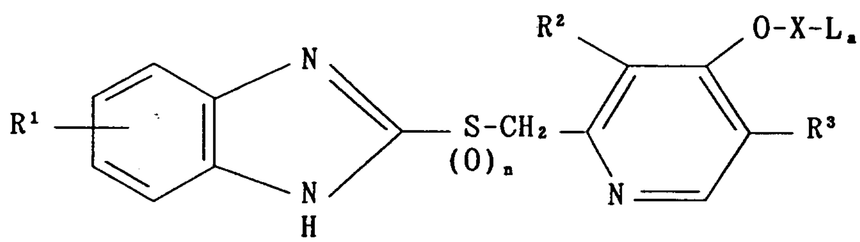

(II)

wherein $R^1$ is hydrogen, halogen, alkyl, alkoxy, alkoxycarbonyl, or haloalkyl, $R^2$ and $R^3$ are the same or different and each is hydrogen, halogen or alkyl, n is 0, 1 or 2, X is single bond or alkylene, and $L_a$ is a group of the formula

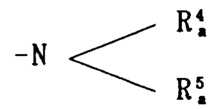

wherein $R_a^4$ is alkyl and $R_a^5$ is furylalkyl, thienylalkyl or pyridylalkyl which may be substituted, or a group of the formula

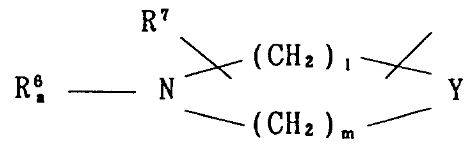

wherein $R_a^6$ is phenylalkyl or substituted phenylalkyl, $R^7$ is hydrogen, halogen, alkyl, alkoxy, haloalkyl, phenyl, substituted phenyl, phenylalkyl, substituted phenylalkyl, heteroarylalkyl, or substituted heteroarylalkyl, Y is methylene, oxygen or sulfur, and l and m are the same or different and each is 0, 1, 2 or 3, or a pharmaceutically acceptable salt thereof.

(4) An agent for the prevention and treatment of disorders in the digestive tract, comprising the compound of the formula (II) or a pharmaceutlcally acceptable salt thereof.

(5) A method for the prevention and treatment of various diseases caused by the bacteria belonging to the genus *Helicobacter*, or for inhibiting recurrence and relapse of ulcer, or for suppressing vomiting, or for the prevention and treatment of non-ulcer dyspepsia, comprising administration of the compound of the formula (I) or a pharmaceutically acceptable salt thereof, or the compound of the formula (II) or a pharmaceutically acceptable salt thereof.

(6) Use of the compound of the formula (I) or a pharmaceutically acceptable salt thereof, or the compound of the formula (II) or a pharmaceutically acceptable salt thereof for the production of an agent for the prevention and treatment of various diseases caused by the bacteria belonging to the genus *Helicobacter*, or an agent for inhibiting recurrence and relapse of ulcer, or a suppressant of vomiting, or an agent for the prevention and treatment of non-ulcer dyspepsia.

In the present specification, halogen means chlorine, bromine, fluorine or iodine; alkyl means alkyl having 1 to 20 carbon atoms such as methyl, ethyl, propyl, isopropyl, butyl, isobutyl, tert-butyl, pentyl, hexyl, octyl, decyl, dodecyl, octadecyl or eicosyl; alkoxy means alkoxy having 1 to 20 carbon atoms such as methoxy, ethoxy, propoxy, isopropoxy, butoxy, isobutoxy, tert-butoxy, pentyloxy, hexyloxy, octyloxy, decyloxy, dodecyloxy, octadecyloxy or elcosyloxy; alkylene means alkylene having 1 to 8 carbon atoms such as methylene, ethylene, methylmethylene, trimethylene, propylene, isopropylidene, tetramethylene, hexamethylene or 2-ethylhexamethylene; alkoxycarbonyl means alkoxycarbonyl having 1 to 20 carbon atoms such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, octyloxycarbonyl, decyloxycarbonyl, dodecyloxycarbonyl, octadecyloxycarbonyl or eicosyloxycarbonyl; haloalkyl means haloalkyl having 1 to 4 carbon atoms such as trifluoromethyl, 2,2,2-tifluoroethyl, 2,3,3-trifluoropropyl, 1,1,2,2-tetrafluoroethyl or 2,2,3,3-tetrafluoropropyl; phenylalkyl means alkyl having 1 to 8 carbon atoms substituted by phenyl such as benzyl, 1-phenylethyl, 2-phenylethyl, 3-phenylpropyl, 4-phenylbutyl, 6-phenylhexyl or 8-phenyloctyl; acyl means alkanoyl having 1 to 5 carbon atoms such as acetyl, propionyl, isopropionyl, butyryl, pivaloyl, valeryl and benzoyl; substituent for substituted phenyl, substituted phenylalkyl, furylalkyl, thienylalkyl and pyridylalkyl means 1 to 3 groups selected from the group consisting of halogen, alkyl, alkoxy, haloalkyl, hydroxyl, nitro and amino; heterocyclic ring formed with the adjacent nitrogen atom, which may be condensed includes 1-pyrrolidinyl, piperidino, 1-piperazinyl, 4-alkyl-1-piperazinyl, 4-aralkyl-1-piperazinyl, 4-substituted aralkyl-1-piperasinyl, 4-alkyl-1-homopiperazinyl, 4-acyl-1-homopiperazinyl, morpholino, thiomorpholino, 2-oxo-1-pyrrolidinyl, 2,4-dioxo-1-pyrrolidinyl, 2-oxo-1-piperidinyl, isoindolin-2-yl, 1,2,3,4-tetrahydroquinolin-1-yl, and 1,2,3,4-tetrahydroisoquinolin-2-yl (these isolndollne ring and 1,2,3,4-tetrahydro(iso)-quinoline ring may be substituted by 1 to 3 substituents optionally selected from the group consisting of halogen, alkyl, alkoxy, haloalkyl, hydroxyl, nitro, amino and oxo); heteroarylalkyl means, for example, 2-thenyl, 3-thenyl, furfuryl, 3-furylmethyl, 2-, 3- or 4-pyridylmethyl, or 2-pyrimidinylmethyl; and the substituent for substituted heteroarylalkyl means 1 to 3 groups selected from the group consisting of halogen, alkyl, alkoxy, haloalkyl, hydroxyl, nitro and amino.

The compound of the formula (I) of the present invention comprises various isomers. The present invention encompasses one of these isomers or a mixture of such isomers.

The pharmaceutically acceptable salt of the compound of the formula (I) includes salts of the formula

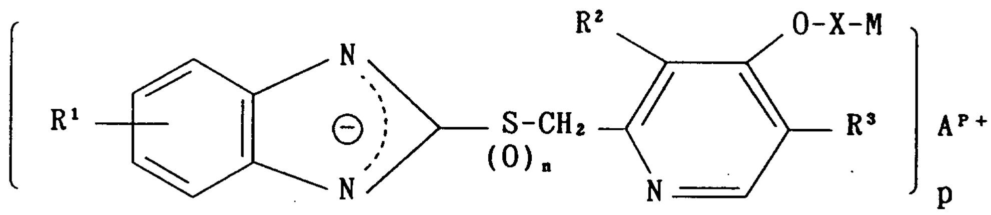

(III)

wherein p is an integer of 1 to 4, $A^{p+}$ is $Li^+$, $Na^+$, $K^+$, $Mg^{2+}$, $Ca^{2+}$, $A\ell^{3+}$, $Ti^{4+}$, $N^+(R)_4$ (wherein R is alkyl having 1 to 4 carbon atoms) or $C^+(NH_2)_3$, M is the definition L or $L_a$, and other symbols are as defined above.

As the compound of the formula (III), particularly preferred are sodium salt, calcium salt, and magnesium salt.

The compound of the present invention exists as hydrates (e.g. semihydrate, monohydrate, sesquihydrate) or as various solvates, all of which are also encompassed in the present invention.

The compound of the formula (I) can be prepared by a known method such as the method disclosed in Japanese Patent Unexamined Publication No. 6270/1989 or International Publication No. WO89/00566.

The compound of the formula (II) can be prepared in a similar manner as in the following, in which a compound of the formula

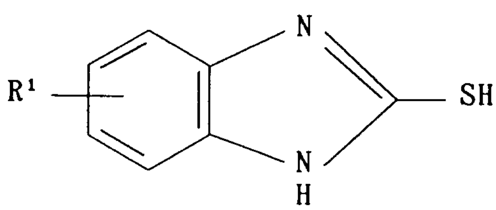

(IV)

wherein $R^1$ is as defined above, is reacted with a compound of the formula

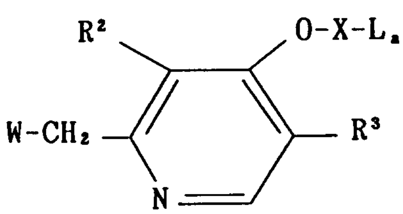

(V)

wherein W is reactive atom or a group such as halogen or sulfonyloxy (e.g. methanesulfonyloxy, benzenesulfonyloxy, p-toluenesulfonyloxy), and other symbols are as defined above, or preferably an acid addition salt thereof, to give a compound of the formula

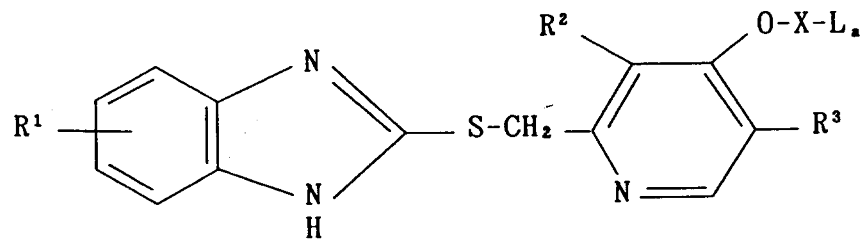

(VI)

wherein each symbol is as defined above, which is then subjected to oxidation.

The reaction of the compound (IV) and compound (V) usually proceeds in an inert solvent such as water, methanol, ethanol, dimethylformamide, or a mixed solvent thereof, preferably aqueous ethanol, in the presence of a base such as sodium hydroxide, potassium hydroxide, sodium hydride, potassium hydride, sodium methoxide, sodium ethoxide, sodium carbonate, potassium carbonate, sodium metal, triethylamine, or pyridine at a temperature between about 0°C and the boiling point of the solvent used, preferably from 20 to 80°C for about 10 minutes to 24 hours, preferably for 30 minutes to 3 hours.

The oxidizing agent to be used for the oxidation includes, for example, perbenzoic acid, m-chloroperbenzoic acid, peracetic acid, trlfluoroperacetic acid, permaleic acid, sodium bromite, sodium hypochlorite, hydrogen peroxide, tert-butyl hydroperoxide, and cumene hydroperoxide. The reaction usually proceeds in an inert solvent such as water, dichloromethane, chloroform, tetrahydrofuran, dioxane, dimethylformamide, or a mixed solvent thereof, in the presence of an organic acid such as formic acid, acetic acid, proplonic acid, butyric acid, maleic acid, fumaric acid, malonic acid, succinic acid, benzoic acid, m-chlorobenzoic acid, p-nitrobenzoic acid, or phthalic acid at a temperature between about -70°C and the boiling point of the solvent used, usually from -50°C to room temperature, preferably from -20°C to 0°C for about 5 minutes to 24 hours, preferably for about 5 minutes to 20 hours; or proceeds in water or an alcohol solvent such as ethanol, methanol, or propanol, in the presence of an alkali such as alkali hydroxide (e.g. sodium hydroxide, potassium hydroxide, calcium hydroxide) at a temperature between about -70°C and the boiling point of the solvent used, usually from -50°C to room temperature, preferably from -20°C to 10°C for about 5 minutes to 20 hours, preferably for 1 hour to 10 hours.

The compound (I) thus synthesized can be separated and purified by known means such as recrystallization and column chromatography.

The optical isomers of the compounds (I) and (II) can be produced by subjecting reaction products to fractional crystallization, or by conducting the aforementioned reactions using the starting materials previously subjected to optical resolution.

The salt compound of the formula (III) can be obtained by reacting a compound (I) or (II) with a base corresponding thereto.

Examples of compound (I), compound (II) and pharmaceutically acceptable salts thereof include the following, to which the present invention is not limited.

Compound 1 : 2-[3-methyl-4-(1-methyl-2-piperidyl)methoxy-2-pyridyl]methylthio-1H-benzimidazole, m.p. 125-126°C

Compound 2 : 2-[3-methyl-4-(3-morpholinopropoxy)-2-pyridyl]methylthio-1H-benzimidazole, m.p. 148-150°C (decomposition)

Compound 3 : 2-[3-methyl-4-(2-piperidinoethoxy)-2-pyridyl]methylthio-1H-benzimidazole, m.p. 105-110°C

Compound 4 : 2-[3-methyl-4-(2-(2-oxo-1-pyrrodinyl)ethoxy)-2-pyridyl]methylthio-1H-benzimidazole, m.p. 64-65°C

Compound 5 : 2-[3-methyl-4-(2-morpholinoethoxy)-2-pyridyl]methylthio-1H-benzimidazole

$^1$H-NMR ($CDCl_3$) : δ (ppm) = 2.28 (s, 3H), 2.50-2.70 and 3.68-3.84 (each m, 8H), 2.88 (t, 2H), 4.20 (t, 2H), 4.40 (s, 2H), 6.76 and 8.34 (each d, 2H), 7.08-7.26 and 7.44-7.60 (each m, 4H)

Compound 6 : 2-[3-methyl-4-(3-piperidinopropoxy)-2-pyridyl]methylthio-1H-benzimidazole, m.p. 114-115°C (decomposition)

Compound 7 : 5-methoxy-2-[3-methyl-4-(2-morpholinoethoxy)-2-pyridyl]methylthio-1H-benzimidazole

$^1$H-NMR ($CDCl_3$) : δ (ppm) = 2.25 (s, 3H), 2.5-2.7 (m, 4H), 2.87 (t, 2H), 3.6-3.85 (m, 4H), 3.85 (s, 3H), 4.2 (t, 2H), 4.4 (s, 2H), 6.7-6.9 (m, 2H), 7.05 (d, 1H), 7.4 (d, 1H), 8.35 (d, 1H)

Compound 8 : 5-methoxy-2-[3-methyl-4-(2-N-benzyl-N-methylamino)ethoxy)-2-pyridyl]methylthio-1H-benzimidazole, m.p. 121-122°C

Compound 9 : 2-[3-methyl-4-(2-(N-methyl-N-(2-phenylethyl)amino)ethoxy)-2-pyridyl]methylthio-1H-benzimidazole

$^1$H-NMR ($CDCl_3$) : δ (ppm) = 2.24 (s, 3H), 2.44 (s, 3H), 2.60-2.84 (m, 4H), 2.92 (t, 2H), 4.12 (t, 2H), 4.39 (s, 2H), 6.72 and 8.32 (each d, 2H), 7.10-7.35 and 7.40-7.60 (each m, 9H)

Compound 10 : 2-[3-methyl-4-(2-(N-methyl-N-(3-phenylpropyl)amino)ethoxy)-2-pyridyl]methylthio-1H-benzimidazole

$^1$H-NMR ($CDCl_3$) : δ (ppm) = 1.64-2.10 (m, 2H), 2.22 (s, 3H), 2.39 (s, 3H), 2.52 and 2.68 (each t, 4H), 2.86 (t, 2H), 4.11 (t, 2H), 4.40 (s, 2H), 6.72 and 8.32 (each d, 2H), 7.04-7.40 and 7.40-7.70 (each m, 9H)

Compound 11 : 2-[3-methyl-4-(2-(N-benzyl)-N-ethylamino)ethoxy)-2-pyridyl]methylthio-1H-benzimidazole

$^1$N-NMR ($CDCl_3$) : δ (ppm) = 1.10 (t, 3H), 2.14 (s, 3H), 2.68 (q, 2H), 2.92 (t, 2H), 3.70 (s, 2H), 4.04 (t, 2H), 4.36 (s, 2H), 6.62 and 8.30 (each d, 2H), 7.00-7.64 (each m, 9H)

Compound 12 : 2-[3-methyl-4-(2-(N-benzyl-N-propylamino)ethoxy)-2-pyridyl]methylthio-1H-benzimidazole

$^1$H-NMR ($CDCl_3$) : δ (ppm) = 0.90 (t, 3H), 1.54 (m, 2H), 2.24 (s, 3H), 2.56 (t, 2H), 2.91 (t, 2H), 3.69 (s, 2H), 4.04 (t, 2H), 4.36 (s, 2H), 6.62 and 8.28 (each d, 2H), 6.96-7.64 (each m, 9H)

Compound 13 : 2-[3-methyl-4-(2-(N-methyl-N-(4-methylbenzyl)amino)ethoxy)-2-pyridyl]methylthio-1H-benzimidazole, m.p. 99-102 °C

Compound 14 : 2-[3-methyl-4-(2-(N-(4-chrolobenzyl)-N-methylamino)ethoxy)-2-pyridyl]methylthio-1H-benzimidazole

Compound 15 : 2-[3-methyl-4-(2-(N-(4-bromobenzyl)-N-methylamino)ethoxy)-2-pyridyl]methylthio-1H-benzimidazole, m.p. 110-112 °C (decomposition)

Compound 16 : 2-[3-methyl-4-(2-(1,2,3,4-tetrahydroisoquinolin-2-yl)ethoxy)-2-pyridyl]methylthio-1H-benzimidazole

Compound 17 : 2-[3-methyl-4-(2-(N-benzyl-N-methylamino)ethoxy)-2-pyridyl]methylsulfinyl-1H-benzimidazole ½ magnesium salt, m.p. 114-120 °C (decomopsition)

Compound 18 : 2-[3-methyl-4-(2-(N-methyl-N-(4-methylbenzyl)amino)ethoxy)-2-pyridyl]methylsulfinyl-1H-benzimidazole ½ magnesium salt, m.p. 127-132 °C (decomposition)

Compound 19 : 2-[3-methyl-4-(2-(N-(4-bromobenzyl)-N-methylaminoethoxy)-2-pyridyl]methylsulfinyl-1H-benzimidazole ½ magnesium-salt, m.p. 119-124 °C (decomposition)

Compound 20 : 2-[3-methyl-4-(2-(1,2,3,4-tetrahydroisoquinolin-2-yl)ethoxy)-2-pyridyl]methylsulfinyl-1H-benzimidazole ½ magnesium salt, m.p. 173-184 °C (decomposition)

Compound 21 : 2-[3-methyl-4-(2-(N-benzyl-N-methylamino)ethoxy)-2-pyridyl]methylsulfonyl-1H-benzimidazole, m.p. 137-139 °C (decomposition)

Compound 22 : 2-[3-methyl-4-(2-N-methyl-N-(4-methylbenzyl)amino)ethoxy)-2-pyridyl]methylsulfonyl-1H-benzimidazole, m.p. 137-139 °C (decomposition)

Compound 23 : 2-[3-methyl-4-(1-benzyl-4-piperidyl)oxy-2-pyridyl]methylthio-1H-benzimidazole

Compound 24 : 2-[3-methyl-4-(1-benzyl-4-piperidyl)oxy-2-pyridyl]methylsulfinyl-1H-benzimidazole½ magnesium salt, dihydrate

Compound 25 : 2-[3-methyl-4-(1-benzyl-2-piperidyl]methoxy-2-pyridyl]methylthio-1H-benzimidazole, m.p. 82-85 °C

Compound 26 : 2-[3-methyl-4-(2-(N-methyl-N-(2-thienylmethyl)amino)ethoxy)-2-pyridyl]methylthio-1H-benzimidazole, m.p. 104-108 °C (decomposition)

compound 27 : 2-[3-methyl-4-(2-(N-methyl-N-(2-thienylmethyl)amino)ethoxy)-2-pyridyl]methylsulfinyl-1H-benzimidazole ½ magnesium salt dihydrate

Compound 28 : 2-[3-methyl-4-(1-benzyl-2-piperidyl)methoxy-2-pyridyl]methylsulfinyl-1H-benzimidazole$^1/_2$magnesium salt 5/2 dihydrate

Compound 29 : 2-[3-methyl-4-(2-(N-methyl-N-(3-thienylmethyl)amino)ethoxy)-2-pyridyl]methylthio-1H-benzimidazole

Compound 30 : 2-[3-methyl-4-(2-(N-methyl-N-(3-thienylmethyl)amino)ethoxy)-2-pyridyl]methylsulfinyl-1H-benzimidazole

Compound 31 : 2-[3-methyl-4-(2-(N-methyl-N-(3-methyl-2-thienylmethyl)amino)ethoxy)-2-pyridyl]-methylthio-1H-benzimidazole

Compound 32 : 2-[3-methyl-4-(2-(N-methyl-N-(3-methyl-2-thienylmethyl)amino)ethoxy)-2-pyridyl]-methylsulfinyl-1H-benzimidazole

Compound 33 : 2-[3-methyl-4-(2-(N-methyl-N-(5-methyl-2-thienylmethyl)amino)ethoxy)-2-pyridyl]-methylthio-1H-benzimidazole

Compound 34 : 2-[3-methyl-4-(2-(N-methyl-N-(5-methyl-2-thienylmethyl)amino)ethoxy)-2-pyridyl]-methylsulfinyl-1H-benzimidazole

Compound 35 : 2-[3-methyl-4-(2-(N-methyl-N-(5-ethyl-2-thienylmethyl)amino)ethoxy)-2-pyridyl]methylthio-1H-benzimidazole

Compound 36 : 2-[3-ethyl-4-(2-(N-methyl-N-(5-ethyl-2-thienylmethyl)amino)ethoxy)-2-pyridyl]-methylsulfinyl-1H-benzimidazole

Compound 37 : 2-[3-methyl-4-(2-(N-methyl-N-(5-bromo-2-thienylmethyl)amino)ethoxy)-2-pyridyl]-methylthio-1H-benzimidazole

Compound 38 : 2-[3-methyl-4-(2-(N-methyl-N-(5-bromo-2-thienylmethyl)amino)ethoxy)-2-pyridyl]-methylsulfinyl-1H-benzimidazole

Compound 39 : 2-[3-methyl-4-(2-(N-methyl-N-(2-furylmethyl)amino)ethoxy)-2-pyridyl]methylthio-1H-benzimidazole

Compound 40 : 2-[3-methyl-4-(2-(N-methyl-N-(2-furylmethyl)amino)ethoxy)-2-pyridyl]methylsulfinyl-1H-benzimidazole

Compound 41 : 2-[3-methyl-4-(2-(N-methyl-N-(3-furylmethyl)amino)ethoxy)-2-pyridyl]methylthio-1H-benzimidazole

Compound 42 : 2-[3-methyl-4-(2-(N-methyl-N-(3-furylmethyl)amino)ethoxy)-2-pyridyl]methylsulfinyl-1H-benzimidazole

Compound 43 : 2-[3-methyl-4-(2-(N-methyl-N-(5-methyl-2-furylmethyl)amino)ethoxy)-2-pridyl]methylthio-1H-benzimidazole

Compound 44 : 2-[3-methyl-4-(2-(N-methyl-N-(5-methyl-2-furylmethyl)amino)ethoxy)-2-pyridyl]-methylsulfinyl-1H-benzimidazole

Compound 45 : 2-[3-methyl-4-(2-(N-methyl-N-(2-pyridylmethyl)amino)ethoxy)-2-pyridyl]methylthio-1H-benzimidazole

Compound 46 : 2-[3-methyl-4-(2-(N-methyl-N-(2-pyridylmethyl)amino)ethoxy)-2-pyridyl]methylsulfinyl-1H-benzimidazole

Compound 47 : 2-[3-methyl-4-(3-methyl-4-(3-methoxypropoxy)-2-pyridyl]methylsulfinyl-1H-benzimidazole sodium salt

The compound (I) shows selective antibacterial activity on the bacteria belonging to the genus *Helicobacter* represented by *Helicobacter pylori*. Accordingly, the compound (I) of the present invention is effective in the prevention and treatment of various diseases caused by the bacteria belonging to the genus *Helicobacter*. That is, the compound of the present invention is usable for the prevention and treatment of various diseases in mammals inclusive of human, which are caused by the bacteria belonging to the genus *Helicobacter*, for inhibiting recurrence and relapse of ulcer, for suppressing vomiting, for the prevention and treatment of non-ulcer dyspepsia, or for the prevention and treatment of tumor. In addition, the novel compound (II) of the present invention has antiulcer activity, gastric acid secretion-suppressing activity, gastrointestinal cell-protecting activity, and antidiarrhea activity besides the aforementioned activity, and is useful for the prevention and treatment of disorders in the digestive tracts such as gastric ulcer, duodenal ulcer, gastritis, diarrhea, and colitis. Also, the compound is characterized by low toxicity and small rise of gastrin value in blood.

When using the compound (I), (II) or a salt thereof as a pharmaceutical, it can be administered generally in the form of a pharmaceutical preparation comprising said compound per se or a pharmaceutically acceptable salt thereof in admixture with pharmaceutically acceptable carrier, upon mixing same with additives such as excipients, carriers, diluents, solubilizing agents, etc. and formulation into the form of capsules, tablets (including sugar-coated tablets and film-coated tablets), granules, injections or transfusions. In the case of oral administration, the dose is about 0.01-20 mg/kg/day, preferably 0.1-4 mg/kg/day for an adult, though it may vary depending on symptoms, age, resistance to drug, etc. of patients.

Experiment 1

Antibacterial activity (*in vitro*) of the active ingredient compounds of the invention against *Helicobacter pylori* CPY-4, CPY-13, CPY-23 and ATCC 43504 were determined by the agar plate dilution method described below.

The test strains were incubated microaerobically at 37°C for 72 hours on agar supplemented with 5% horse serum and were diluted with Brucella broth (BBL) to about $10^6$ CFU/ml. Each bacterial suspension was applied to the Brucella-blood agar plate containing twofold serial dilutions of the test compounds by a multipoint inoculator (Microplanter). The plates were incubated at 37°C for 2 days in the presence of 10% $CO_2$. The MIC was defined. The results are shown in Table 1.

Table 1

| Test compound | CPY-4 | MIC (μg/ml) CPY-13 | CPY-23 | ATCC 43504 |
|---|---|---|---|---|
| 1 | 1.56 | 1.56 | 3.13 | 3.13 |
| 2 | 0.39 | 0.39 | 0.39 | 0.78 |
| 3 | 1.56 | 1.56 | 1.56 | 6.25 |
| 4 | 0.78 | 0.78 | 0.78 | 3.13 |
| 5 | 0.012 | 0.012 | 0.05 | 0.05 |
| 6 | 3.13 | 3.13 | 3.13 | 6.25 |
| 7 | 0.39 | 0.39 | 0.39 | 0.78 |
| 8 | 0.39 | 0.39 | 0.39 | 0.39 |
| 9 | 0.10 | 0.10 | 0.20 | 0.20 |
| 10 | 0.20 | 0.20 | 0.20 | 0.20 |
| 11 | 0.025 | 0.05 | 0.10 | 0.10 |
| 12 | 0.20 | 0.39 | 0.39 | 0.78 |
| 13 | 0.05 | 0.10 | 0.39 | 0.20 |
| 14 | 0.025 | 0.10 | 0.20 | 0.10 |
| 15 | 0.10 | 0.10 | 0.05 | 0.10 |
| 16 | 0.012 | 0.012 | 0.05 | 0.012 |
| 17 | 0.025 | 0.025 | 0.10 | 0.05 |
| 18 | 0.10 | 0.10 | 0.10 | 0.20 |
| 19 | 0.05 | 0.10 | 0.05 | 0.10 |
| 20 | 0.012 | 0.012 | 0.012 | 0.012 |
| 21 | 0.39 | 0.78 | 0.39 | 1.56 |
| 22 | 0.78 | 0.78 | 0.78 | 1.56 |
| 23 | 0.05 | 0.05 | 0.20 | 0.20 |
| 24 | 0.05 | 0.05 | 0.20 | 0.10 |
| 25 | 0.39 | 0.39 | 0.39 | 0.39 |
| 26 | 0.05 | 0.025 | 0.20 | 0.10 |
| 27 | 0.05 | 0.05 | 0.10 | 0.10 |
| 28 | 0.39 | 0.78 | 0.39 | 0.78 |

Experiment 2

The test strains were preincubated in Muller-Hinton broth and were diluted with the same broth to about $10^6$ CFU/ml. 5 μl of an inoculum was inoculated onto an agar containing twofold serial dilutions of the test compound. The plates were incubated at 37°C for 18 hours. The minimum Inhibitory concentration (MIC) was defined as the lowest concentration of the test compound inhibiting formation of bacterial colony. The results are shown in Table 2.

Table 2

| Test bacteria | MIC (μg/ml) | |
|---|---|---|
| | Compound 17 | Compound 20 |
| *Staphylococcus aureus* FDA 209P | 100 | >100 |
| *Staphylococcus epidermidis* ATCC 12228 | 100 | 100 |
| *Enterococcus faecalis* LS-101 | >100 | >100 |
| *Bacillus subtilis* PCI 219 | >100 | >100 |
| *Escherichia coli* NIHJ JC-2 | >100 | >100 |
| *Klebsiella pneumoniae* DT | >100 | >100 |
| *Proteus vulgaris* IFO 3988 | >100 | >100 |
| *Acinetobacter calcoaceticus* IFO 13006 | >100 | >100 |
| *Pseudomonas aeruginosa* IFO 12582 | >100 | >100 |
| *Candida albicans* IFO 1060 | >100 | >100 |

As is eveident from Table 2, the compound of the formula (I) had no effect on Gram-positive bacteria, Gram-negative bacteria and fungi.

Experiment 3

Acid secretion in the gastric lumen-perfused rats

Experiments were performed according to the method described by Ghosh et al. (Br. J. Pharmacol. *13*. 54, 1958). Wistar rats were deprived of food for 20 hours and anesthetized with urethane (1.5 g/kg, s.c.). A polyethylene tube canula for introducing perfusion liquid and another tube for draining perfusate were inserted into the stomach. The stomach was perfused at a flow rate of 7 ml/min with warmed saline (37°C) and perfusate was collected for acid measurement. Acid secretion induced by histamine hydrochloride (1 mg/kg, i.v.) every hour was measured by antonomic titration of the perfusate to pH 7.0. Test compounds were administered intravenously 5 min before histamine hydrochloride injection. The results are shown in Table 3 as $ED_{50}$ values; the doses caused 50% inhibition of gastric acid secretion.

Table 3

| Test compound (Example No.) | $ED_{50}$ (mg/kg, i.v.) |
|---|---|
| 2 | 1.5 |
| 5 | 0.9 |
| 6 | 1.7 |

The present invention is hereinbelow detailedly described by illustrating Examples and Formulation Examples. It should be understood that the present invention is not limited to them.

Example 1

2-Chloromethyl-3-methyl-4-[(1-benzyl-4-piperidyl)oxy]pyridine dihydrochloride (8.7 g) was added to 120 ml of ethanol containing 2-mercaptobenzimidazole (3.6 g) and 13.5% sodium hydroxide (23 ml), and the mixture was stirred at room temperature for 1 hour. After the completion of the reaction, ethanol was distilled off, and water was added to the residue. The precipitated crystals were collected by filtration, and recrystallized from ethyl acetate to give 2-[3-methyl-4-(1-benzyl-4-piperidyl)oxy-2-pyridyl]methylthio-1H-benzimidazole (Compound 23), m.p. 148 °C.

Example 2

To 2-[3-methyl-4-(1-benzyl-4-piperidyl)oxy-2-pyridyl]methylthio-1H-benzimidazole (7.48 g) in chloroform (150 ml) was added m-chlorobenzoic acid, and the mixture was stirred at room temperature for 20 minutes.

After cooling the mixture to -20 °C, 80% m-chloroperbenzoic acid (4.36 g) was added, and the mixture was stirred for 30 minutes. Then, ammonia gas was passed through, and the resultant precipitation was filtered off. The filtrate was concentrated under reduced pressure, and the residue was subjected to alumina column chromatography and eluted with chloroform containing 1% ethanolic ammonia to give a sulfinyl compound in amorphous powder.

The obtained sulfinyl compound was dissolved in ethanol, and an aqueous solution of 1.9% sodium hydroxide (10 ml) was added thereto. After stirring for 10 minutes, the mixture was concentrated under reduced pressure. Deionized water (100 ml) was added to the residue, then magnesium chloride was added while stirring, and the precipitated crystals were collected by filtration to give 2-[3-methyl-4-(1-benzyl-4-piperidyl)oxy-2-pyridyl]methylsulfinyl-1H-benzimidazole $\frac{1}{2}$ magnesium salt dihydrate (Compound 24).

| Elemental analysis for $C_{26}H_{28}N_4O_2S$ $\frac{1}{2}$Mg $2H_2O$ | | | |
|---|---|---|---|
| Calculated<br>Found | C 61.50;<br>C 61.04; | H 6.15;<br>H 5.92; | N 11.03<br>N 10.70 |

Examples 3-25

In the same manner as above, the following compounds can be obtained.

(3) 2-[3-methyl-4-(1-benzyl-2-piperidyl)methoxy-2-pyridyl]methylthio-1H-benzimidazole (Compound 25), m.p. 82-85 °C

(4) 2-[3-methyl-4-(2-(N-methyl-N-(2-thienylmethyl)amino)ethoxy-2-pyridyl]methylthio-1H-benzimidazole (Compound 26), m.p. 104-108 °C (decomposition)

(5) 2-[3-methyl-4-(2-(N-methyl-N-(2-thienylmethyl)amino)ethoxy-2-pyridyl]methylsulfinyl-1H-benzimidazole $\frac{1}{2}$ magnesium salt dihydrate (Compound 27)

| Elemental analysis for $C_{22}H_{24}N_4O_2S_2$ $\frac{1}{2}$Mg $2H_2O$ | | | |
|---|---|---|---|
| Calculated<br>Found | C 54.22;<br>C 54.17; | H 5.58;<br>H 5.22; | N 11.49<br>N 11.45 |

(6) 2-[3-methyl-4-(1-benzyl-2-piperidyl)methoxy-2-pyridyl]methylsulfinyl-1H-benzimidazole$\frac{1}{2}$ magnesium salt 5/2 hydrate (Compound 28)

| Elemental analysis for $C_{27}H_{30}N_4O_2S$ $\frac{1}{2}$Mg $5/2H_2O$ | | | |
|---|---|---|---|
| Calculated<br>Found | C 61.10;<br>C 61.40; | H 6.45;<br>H 6.13; | N 10.56<br>N 10.32 |

(7) 2-[3-methyl-4-(2-(N-methyl-N-(3-thienylmethyl)amino)ethoxy-2-pyridyl]methylthio-1H-benzimidazole (Compound 29)

(8) 2-[3-methyl-4-(2-(N-methyl-N-(3-thienylmethyl)amino)ethoxy-2-pyridyl]methylsulfinyl-1H-benzimidazole (Compound 30)

(9) 2-[3-methyl-4-(2-(N-methyl-N-(3-methyl-2-thienylmethyl)amino)ethoxy-2-pyridyl]methylthio-1H-benzimidazole (Compound 31)

(10) 2-[3-methyl-4-(2-(N-methyl-N-(3-methyl-2-thienylmethyl)amino)ethoxy-2-pyridyl]methylsulfinyl-1H-benzimidazole (Compound 32)

(11) 2-[3-methyl-4-(2-(N-methyl-N-(5-methyl-2-thienylmethyl)amino)ethoxy-2-pyridyl]methylthio-1H-benzimidazole (Compound 33)

(12) 2-[3-methyl-4-(2-(N-methyl-N-(5-methyl-2-thienylmethyl)amino)ethoxy-2-pyridyl]metfiylsulfinyl-1H-benzimidazole (Compound 34)

(13) 2-[3-methyl-4-(2-(N-methyl-N-(5-ethyl-2-thienylmethyl)amino)ethoxy-2-pyridyl]methylthio-1H-benzimidazole (Compound 35)

(14) 2-[3-methyl-4-(2-(N-methyl-N-(5-ethyl-2-thienylmethyl)amino)ethoxy-2-pyridyl]methylsulfinyl-1H-benzimidazole (Compound 36)

(15) 2-[3-methyl-4-(2-(N-methyl-N-(5-bromo-2-thienylmethyl)amino)ethoxy)-2-pyridyl]methylthio-1H-benzimidazole (Compound 37)
(16) 2-[3-methyl-4-(2-(N-methyl-N-(5-bromo-2-thienylmethyl)amino)ethoxy)-2-pyridyl]methylsulfinyl-1H-benzimidazole (Compound 38)
(17) 2-[3-methyl-4-(2-(N-methyl-N-(2-furylmethyl)amino)ethoxy)-2-pyridyl]methylthio-1H-benzimidazole (Compound 39)
(18) 2-[3-methyl-4-(2-(N-methyl-N-(2-furylmethyl)amino)ethoxy)-2-pyridyl]methylsulfinyl-1H-benzimidazole (Compound 40)
(19) 2-[3-methyl-4-(2-(N-methyl-N-(3-furylmethyl)amino)ethoxy)-2-pyridyl]methylthio-1H-benzimidazole (Compound 41)
(20) 2-[3-methyl-4-(2-(N-methyl-N-(3-furylmethyl)amino)ethoxy)-2-pyridyl]methylsulfinyl-1H-benzimidazole (Compound 42)
(21) 2-[3-methyl-4-(2-(N-methyl-N-(5-methyl-2-furylmethyl)amino)ethoxy)-2-pyrldyl]methylthio-1H-benzimidazole (Compound 43)
(22) 2-[3-methyl-4-(2-(N-methyl-N-(5-methyl-2-furylmethyl)amino)ethoxy)-2-pyridyl]methylsulfinyl-1H-benzimidazole (Compound 44)
(23) 2-[3-methyl-4-(2-(N-methyl-N-(2-pyridylmethyl)amino)ethoxy)-2-pyridyl]methylthio-1H-benzimidazole (Compound 45)
(24) 2-[3-methyl-4-(2-(N-methyl-N-(2-pyridylmethyl)amino)ethoxy)-2-pyridyl]methylsulfinyl-1H-benzimidazole (Compound 46)
(25) 2-[3-methyl-4-(3-methyl-(3-propoxy)-2-pyridyl]methylsulfinyl-1H-benzimidazole sodium salt (Compound 47)

Formulation Examples

Tablets

The tablets containing 20 mg of the active ingredient can be prepared according to the following formulation.

| | |
|---|---|
| Compound of Example 1 | 20 mg |
| Corn starch | 15 mg |
| Lactose | 57 mg |
| Microcrystalline cellulose | 25 mg |
| Magnesium stearate | 3 mg |
| | 120 mg |

Capsules

The capsules containing 20 mg of the active ingredient can be prepared according to the following formulation.

| | |
|---|---|
| Compound of Example 1 | 20 mg |
| Corn starch | 30 mg |
| Lactose | 63 mg |
| Hydroxypropylcellulose | 6 mg |
| Magnesium stearate | 1 mg |
| | 120 mg |

While the present invention has been adequately and sufficiently described by the foregoing specification and examples contained in the specification, it should be understood that the invention is susceptible to various modifications and alternative forms within the spirit and scope of the invention.

## Claims

1. An agent for the prevention and treatment of various diseases caused by the bacteria belonging to the genus *Helicobacter*, comprising a pyridine compound of the formula

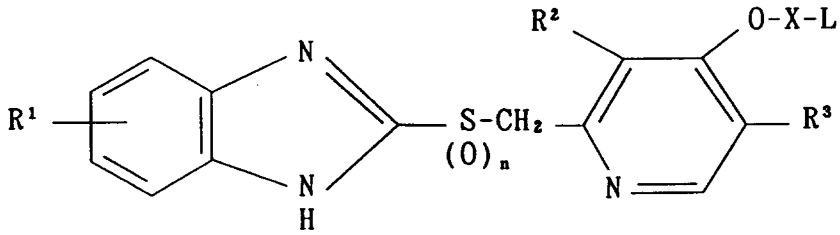

wherein $R^1$ is hydrogen, halogen, alkyl, alkoxy, alkoxycarbonyl, or haloalkyl, $R^2$ and $R^3$ are the same or different and each is hydrogen, halogen or alkyl, n is 0, 1 or 2, X is single bond or alkylene, and L is alkoxy, a group of the formula

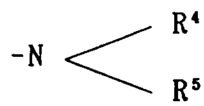

wherein $R^4$ and $R^5$ are the same or different, and each is hydrogen, alkyl, acyl, phenyl, substituted phenyl, phenylalkyl, substituted phenylalkyl, or furylalkyl, thienylalkyl or pyridylalkyl which may be substituted, or $R^4$ and $R^5$ together with the adjacent nitrogen atom form a heterocyclic ring which may be condensed, or a group of the formula

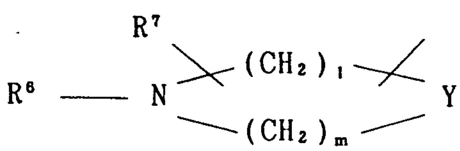

wherein $R^6$ is hydrogen, alkyl, acyl, phenylalkyl, or substituted phenylalkyl, $R^7$ is hydrogen, halogen, alkyl, alkoxy, haloalkyl, phenyl, substituted phenyl, phenylalkyl, substituted phenylalkyl, heteroarylalkyl, or substituted heteroarylalkyl, Y is methylene, oxygen or sulfur, and l and m are the same or different and each is 0, 1, 2 or 3, or a pharmaceutically acceptable salt thereof.

2. An agent for inhibiting recurrence and relapse of ulcer, a suppressant of vomiting, or an agent for the prevention and treatment of non-ulcer dyspepsia, comprising a pyridine compound of the formula

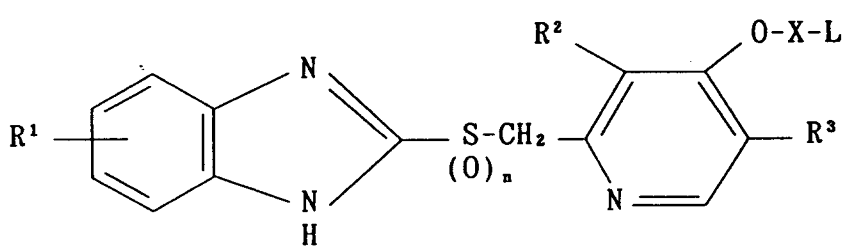

wherein $R^1$ is hydrogen, halogen, alkyl, alkoxy, alkoxycarbonyl, or haloalkyl, $R^2$ and $R^3$ are the same or different and each is hydrogen, halogen or alkyl, n is 0, 1 or 2, X is single bond or alkylene, and L is alkoxy, a group of the formula

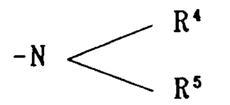

wherein $R^4$ and $R^5$ are the same or different, and each is hydrogen, alkyl, acyl, phenyl, substituted phenyl, phenylalkyl, substituted phenylalkyl, or furylalkyl, thienylalkyl or pyridylalkyl which may be substituted, or $R^4$ and $R^5$ together with the adjacent nitrogen atom form a heterocyclic ring which may be condensed, or a group of the formula

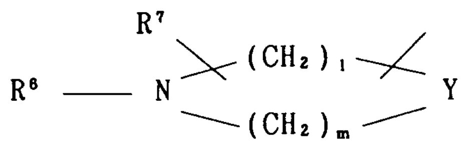

wherein $R^6$ is hydrogen, alkyl, acyl, phenylalkyl, substituted phenylalkyl, $R^7$ is hydrogen, halogen, alkyl, alkoxy, haloalkyl, phenyl, substituted phenyl, phenylalkyl, substituted phenylalkyl, heteroarylalkyl, or substituted heteroarylalkyl, Y is methylene, oxygen or sulfur, and l and m are the same or different and each is 0, 1, 2 or 3, or a pharmaceutically acceptable salt thereof.

**3.** A pyridine compound of the formula

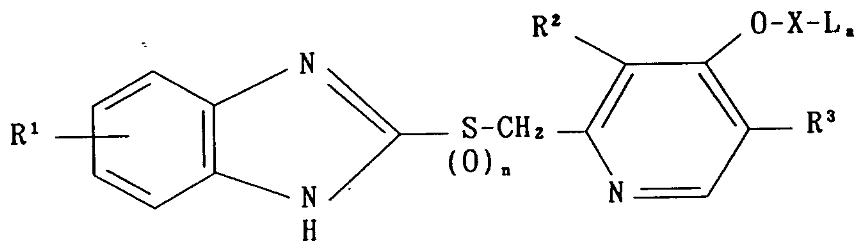

wherein $R^1$ is hydrogen, halogen, alkyl, alkoxy, alkoxycarbonyl, or haloalkyl, $R^2$ and $R^3$ are the same or different and each is hydrogen, halogen or alkyl, n is 0, 1 or 2, X is single bond or alkylene, and $L_a$ is a group of the formula

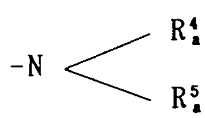

wherein $R_a^4$ is alkyl and $R_a^5$ is furylalkyl, thienylalkyl or pyridylalkyl which may be substituted, or a group of the formula

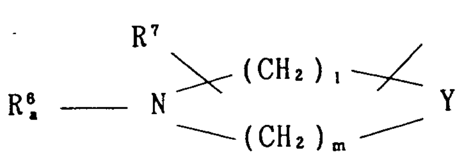

wherein $R_a^6$ is phenylalkyl or substituted phenylalkyl, $R^7$ is hydrogen, halogen, alkyl, alkoxy, haloalkyl, phenyl, substituted phenyl, phenylalkyl, substituted phenylalkyl, heteroarylalkyl, or substituted heteroarylalkyl, Y is methylene, oxygen or sulfur, and l and m are the same or different and each is 0, 1, 2 or 3, or a pharmaceutically acceptable salt thereof.

**4.** An agent for the prevention and treatment of disorders in the digestive tract, comprising the compound of Claim 3.

**5.** The agent for the prevention and treatment of disorders in the digestive tract, according to Claim 4, wherein the agent for the prevention and treatment is an antiulcer.

**6.** A method for the prevention and treatment of various diseases caused by the bacteria belonging to the genus *Helicobacter*, comprising administration of the compound of Claim 1 or Claim 3.

**7.** A method for inhibiting recurrence and relapse of ulcer, or for suppressing vomiting, or for the prevention and treatment of non-ulcer dyspepsia, comprising administration of the compound of Claim 1 or Claim 3.

**8.** Use of the pyridine compound of Claim 1 or Claim 3, or a pharmaceutically acceptable salt thereof for the production of an agent for the prevention and treatment of various diseases caused by the bacteria belonging to the genus *Helicobacter.*

**9.** Use of the pyridine compound of Claim 1 or Claim 3, or a pharmaceutically acceptable salt thereof for the production of an agent for inhibiting recurrence and relapse of ulcer, or a suppressant of vomiting, or an agent for the prevention and treatment of non-ulcer dyspepsia.

# INTERNATIONAL SEARCH REPORT

International Application No PCT/JP91/00037

**I. CLASSIFICATION OF SUBJECT MATTER** (if several classification symbols apply, indicate all)

According to International Patent Classification (IPC) or to both National Classification and IPC

Int. $Cl^5$ C07D401/12, 401/14, 405/14, 409/14, 413/14, 417/14, A61K31/44, 31/535, 31/54

**II. FIELDS SEARCHED**

Minimum Documentation Searched

| Classification System | Classification Symbols |
|---|---|
| IPC | C07D401/12-401/14, 405/14, 409/14, 413/14, 417/14, A61K31/44, 31/535, 31/54 |

Documentation Searched other than Minimum Documentation to the Extent that such Documents are Included in the Fields Searched

**III. DOCUMENTS CONSIDERED TO BE RELEVANT**

| Category * | Citation of Document, with indication, where appropriate, of the relevant passages | Relevant to Claim No. |
|---|---|---|
| X | JP, A, 59-181277 (Aktiebolaget Hassle), October 15, 1984 (15. 10. 84), Columns 1 to 11 & DE, A, 3404610, FR, A, 2543551 | 1, 2, 3 |
| X | JP, A, 2-22225 (Eisai Co., Ltd.), January 25, 1990 (25. 01. 90), Column 1 | 1, 2, 3 |
| X | JP, A, 1-6270 (Eisai Co., Ltd.), January 10, 1989 (10. 01. 89), & EP, A, 268956 | 1, 2, 3 |
| X | JP, A, 2-22273 (Yoshitomi Pharmaceutical Co., Ltd.), January 25, 1990 (25. 01. 90), Columns 1 to 4 & WO, A, 8900566 | 1, 2, 3 |
| A | JP, A, 61-85383 (Banyu Pharmaceutical Co., Ltd.), April 30, 1986 (30. 04. 86) | 1, 2, 3 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 5, 1991 (05. 04. 91) | April 15, 1991 (15. 04. 91) |
| **International Searching Authority** | **Signature of Authorized Officer** |
| Japanese Patent Office | |